# EUROPÄISCHE PATENTANMELDUNG

(11) **EP 1 179 525 A1**
(43) Veröffentlichungstag der Anmeldung: **13.02.2002**
(21) Anmeldenummer: 01117084.2
(22) Anmeldetag: 13.07.2001
(51) Int. Cl.: C07C 231/10, C07D 295/18

(54) **Verfahren zur Herstellung von Carbonsäureamiden durch Oxidation von aldehyden in Gegenwart von aminen**

(30) Priorität: 11.08.2000 DE 10039247
(71) Anmelder: Degussa Aktiengesellschaft, 40474 Düsseldorf (DE)
(72) Erfinder: Riermeier, Thomas, Dr., 65439 Flörsheim (DE); Beller, Matthias, Prof.Dr., 18119 Rostock (DE); Tillack, Annegret, Dr., 18146 Rostock (DE); Rudloff, Ivo, Dr., Southampton SO 17 3 TL (GB)
(74) Vertreter: Ackermann, Joachim, Dr.

(57) **Zusammenfassung**

Verfahren zur Herstellung von mono-, bi- oder/und polyfunktionellen Amiden der Formeln (Ia) oder/und (Ib),

R¹-CO-NR²R³ (Ia)

R⁴R⁵N-CO-R⁶-CO- NR²R³ (Ib)

aus Aldehyden und Aminen in Gegenwart eines Übergangsmetallkatalysators und einem Oxidationsmittel.

## Beschreibung

Die vorliegende Erfindung betrifft ein Verfahren zur Herstellung von aliphatischen und aromatischen Carbonsäureamiden aus Aldehyden und Aminen in Gegenwart eines Übergangsmetallkatalysators und einem Oxidationsmittel.

Carbonsäureamide haben technische Bedeutung als Feinchemikalien, sie sind Ausgangsprodukte für Polymere (Polyamide), Funktionsmaterialien, Agrochemikalien und Pharmawirkstoffe.

Generell werden Carbonsäureamide durch Reaktion von aktivierten Carbonsäurederivaten wie beispielsweise Carbonsäureestern, Carbonsäureanhydriden, Carbonsäurehalogeniden, etc. und Aminen in Gegenwart eines Katalysators oder eines Kondensationsreagenz hergestellt (D. Döpp, H. Döpp, In Houben-Weyl Methoden der organischen Chemie Band E5 ; J. Falbe, Ed; Georg Thieme Verlag: Stuttgart, 1985, S. 934. (b) In Comprehensive Organic Transformations: a guide to functional group preparations; R. C. Larock, Ed; VCH: Weinheim, 1989, S. 885, 994.)
Bei diesem Verfahren werden stöchiometrische Mengen von Nebenprodukten - häufig Salzabfälle - gebildet. Amidsynthesen, die diese ökologischen Probleme vermeiden, sind heterogen-katalysierte Aminierungen von Carbonsäuren. Allerdings verlaufen diese Reaktionen unter drastischen Reaktionsbedingungen (sehr hohe Reaktionstemperatur), so daß Carbonsäureamide mit sensitiven funktionellen Gruppen nicht selektiv darstellbar sind. (Beckwitch in Zabicky, The Chemstry of Amides, Wiley, NY, 1970,105-109)
Ein weiteres Problem von Amidsynthesen ist zum Teil die Zugänglichkeit des entsprechenden Carbonsäurederivats.

Eine alternative Amidsyntheseroute geht von kostengünstigen Aldehyden aus (Y.Tamura, Y.Yamada, Z. Yoshida, Synthesis 1983, 474). Tamura und Mitarbeiter beschreiben so die Synthese von Amiden aus Aldehyden in Gegenwart von Palladiumkatalysatoren. Als Oxidationsmittel werden Arylbromide eingesetzt. Dieses Verfahren hat den Nachteil, daß stöchiometrische Mengen an teurem Brombenzol eingesetzt werden müssen und daß stöchiometrische Mengen an Bromabfällen entstehen.
Andere bekannte Verfahren Amide aus Aldehyden herzustellen (K. Nakagawa, H. Onoue, K. Minami, Chem. Commun. 1966, 4319. S. Fukuoka, M. Ryang, S. Tsutsumi, J. Org. Chem. 1971, 36, 2721) benötigen stöchiometrische Mengen an Übergangsmetallverbindungen was diese Verfahren sowohl ökonomisch als auch ökologisch unattraktiv macht.
Naota beschreibt eine Rutheniumkatalysierte Variante dieser Reaktion (Naota, Synlett, 1991, 693). Hierbei wird schwer zugängliches und nur sehr aufwendig recyclierbares Benzylidenaceton als Oxidationsmittel verwendet.
Ferner ist in DE 2953007 C1 die Verwendung Heterogener Katalysatoren für diese Umsetzung beschrieben , wobei die erzielten Ausbeuten gering sind und industriellen Anforderungen nicht genügen.

Aus den genannten Gründen bestand ein Bedarf nach einem neuen Verfahren, das Amide möglichst einfach, kostengünstig und großtechnisch einsetzbar aus Aldehyden und Aminen zugänglich macht.

Diese Aufgabe wird gelöst durch ein Verfahren zur Herstellung von mono-, bi- oder/und polyfunktionellen Amiden der Formeln (la) oder/und (Ib),

R¹-CO-NR²R³ (Ia)

R⁴R⁵N-CO-R⁶-CO- NR²R³ (Ib)

worin R¹ für Wasserstoff, einen (C₁-C₁₈)-Alkylrest, (C₂-C₁₈)-Alkenylrest, (C₂-C₁₈)-Alkinylrest, die verzweigt, linear oder cyclisch sein können, sowie für einen bis zu 14 C-Atome enthaltenden aromatischen Arylrest oder Heteroarylrest, enthaltend ein bis vier Heteroatome aus der Gruppe N, O oder S, steht,
wobei der Alkylrest und/oder Alkenylrest und/oder Alkinylrest und/oder Arylrest neben Wasserstoffatomen und der Amidgruppe bis zu fünf weitere Substituenten tragen kann, die unabhängig voneinander (C₁-C₈)-Alkyl, O-Alkyl-(C₁-C₈), O-Aryl, OCO-Alkyl-(C₁-C₈), OCO-Aryl, O-Phenyl, Phenyl, Aryl, Fluor, Chlor, Brom, lod, OH, NO₂, SiAlkyl₃-(C₁-C₈), CN, COOH, SO₃H, NH-Alkyl-(C₁-C₈), NH-Aryl, N-Alkyl₂-(C₁-C₈), N-Aryl₂, SO₂-Alkyl-(C₁-C₆), SO₂-Aryl, SO-Alkyl-(C₁-C₆), CF₃, NHCO-Alkyl-(C₁-C₄), COO-Alkyl-(C₁-C₈), COOAryl, CONH₂, CO-Alkyl-(C₁-C₈), CO-Aryl, NHCOH, NHCOO-Alkyl-(C₁-C₄), CO-Phenyl, COO-Phenyl, CHCH-CO₂-Alkyl-(C₁-C₈), PO-Phenyl₂, POAlkyl₂-(C₁-C₄), PO₃H₂, PO(O-Alkyl-(C₁-C₆))₂, SO₃-Alkyl-(C₁-C₄) bedeuten,
wobei der Arylrest für einen fünf-, sechs- oder siebengliedrigen aromatischen oder heteroaromatischen Ring mit ein bis vier Heteroatomen aus der Gruppe N, O und S steht und wobei an diesen Ring weitere aromatische, heteroaromatische oder/und aliphatische Ringe mit 4 bis 16 Kohlenstoffatomen ankondensiert sein können, wobei 1 bis 8 Kohlenstoffatome durch Heteroatome aus der Gruppe N, O, S ersetzt sein können, und worin
R² bis R⁵ unabhängig voneinander Wasserstoff, Alkyl-(C₁-C₁₈), Aryl, bedeuten,
wobei Alkyl und Aryl die oben genannte Bedeutung haben und bis zu 5 der oben genannten Substituenten neben Wasserstoff besitzen können,
und worin
R⁶ für einen (C₁-C₁₈)-Alkylenrest, der verzweigt, linear und/oder cyclisch sein kann, sowie für einen bis zu 14 C-Atome enthaltenden aromatischen Arylenrest steht, wobei der Alkylenrest und/oder Arylenrest neben Wasserstoffatomen und den Amidgruppen bis zu fünf weitere Substituenten tragen kann, die unabhängig voneinander (C₁-C₈)-Alkyl, O-Alkyl-(C₁-C₈), O-Aryl, OCO-Alkyl-(C₁-C₈), OCO-Aryl, O-Phenyl, Phenyl, Aryl, Fluor, Chlor, Brom, lod, OH, NO₂, SiAlkyl₃-(C₁-C₈), CN, COOH, SO₃H, NH-Alkyl-(C₁-C₈), NH-Aryl, N-Alkyl₂-(C₁-C₈), N-Aryl₂, SO₂-Alkyl(C₁-C₆), SO₂-Aryl, SO-Alkyl-(C₁-C₆), CF₃, NHCO-Alkyl-(C₁-C₄), COO-Alkyl-(C₁-C₈), COOAryl, CONH₂, CO-Alkyl-(C₁-C₈), CO-Aryl, NHCOH, NHCOO-Alkyl-(C₁-C₄), CO-Phenyl, COO-Phenyl, CHCH-CO₂-Alkyl-(C₁-C₈), PO-Phenyl₂, POAlkyl₂-(C₁-C₄), PO₃H₂, PO(O-Alkyl-(C₁-C₆))₂, SO₃-Alkyl-(C₁-C₄) bedeuten, wobei der Arylrest auch für einen fünf-, sechs- oder siebengliedrigen aromatischen oder heteroaromatischen Ring steht, wobei der heteroaromatische Ring ein bis vier Heteroatomen aus der Gruppe N, O, S enthalten kann, wobei an diesen Ring weitere aromatische, heteroaromatische oder/und aliphatische Ringe mit 4 bis 16 Kohlenstoffatomen ankondensiert sein können, wobei 1 bis 8 Kohlenstoffatome durch Heteroatome aus der Gruppe N, O, S ersetzt sein können,
durch Umsetzung von Aldehyden oder/und Dialdehyden der allgemeinen Formeln (lla) oder/und (IIb)

R¹-CHO (IIa)

OHC-R⁶-CHO (IIb)

mit Aminen der allgemeinen Formel (IIIa,b),

R⁴R⁵NH (IIIa)

HNR²R³ (IIIb)

worin bei den Formeln des Typs II und III R¹ bis R⁶ die zuvor zu den Formeln des Typs (I) angegebene Bedeutung besitzen,
in Gegenwart eines Übergangsmetallkatalysators der VIII. Nebengruppe und einem Oxidationsmittel.

Besonders bevorzugt ist ein Verfahren, bei dem Alkylamide, bevorzugt Dialkylamide der Formel (I) hergestellt werden, worin die Reste R² bis R⁵ für (C₁-C₈)-Alkyl oder die beschriebenen substituierten (C₁-C₈)-Alkyle stehen.

Das erfindungsgemäße Verfahren hat sich insbesondere für die Herstellung von substituierten Benzoesäureamiden, Fettamiden und niederen aliphatischen Amiden bewährt.

Als Lösungsmittel des Verfahrens finden im allgemeinen inerte organische Lösungsmittel Verwendung. Besonders geeignet sind aliphatische Ether, aromatische und/oder aliphatische Kohlenwasserstoffe und Ester sowie deren Gemische. Daneben kann die Reaktion auch in Alkoholen, Wasser, Aminen oder ohne Lösungsmittel duchgeführt werden.

Die Reaktion läuft vorzugsweise bei Temperaturen von 20 bis 200 °C ab; in vielen Fällen hat es sich bewährt, bei Temperaturen von 60 bis 180 °C, bevorzugt 80 bis 140 °C, zu arbeiten. Die Reaktion kann unter Druck durchgeführt werden, insbesondere wenn niedrig siedende Aldehyde bzw. Amine eingesetzt werden.

Häufig ist es bei den Reaktionen vorteilhaft, dem Reaktionsgemisch eine Base als Co-Katalysator zuzusetzen. Dafür geeignet sind Trialkylamine, die alicyclisch oder offenkettig sein können, oder Alkali- oder Erdalkalisalze aliphatischer oder aromatischer Carbonsäuren, wie Acetate, Propionate, Benzoate bzw. entsprechende Carbonate, Hydrogencarbonate, Phosphate, Hydrogenphosphate oder Hydroxide bevorzugt von Lithium, Natrium, Kalium, Calcium, Magnesium, Cäsium oder Mischungen solcher Basen.

Die eingesetzten Übergangsmetallkatalysatoren sind Metallverbindungen der VIII.Nebengruppe wie Rh, Pd, Ir, Ru, Co, Pt, etc. Bevorzugt sind als Übergangsmetallkatalysatoren Rh, Ir, Pt und Ru. Besonders bevorzugt sind Rhodiumkatalysatoren. Die Katalysatoren können als homogene Metallkomplexe bzw. als heterogene Katalysatoren eingesetzt werden.

Als Übergangsmetallkatalysatoren können beispielsweise eingesetzt werden:
Rhodium(I)-bis-(1,5-cyclooctadien)-tetrafluoroborat, Rhodium(I)-(1,5-cyclooctadien)-acetylacetonat, Rhodium(I)-(1c,5c-cyclooctadien)-chlorid dimer, Palladium(II)acetat, Palladium(II)chlorid, Lithiumtetrachloropalladat, Palladium(II)acetylacetonat, Palladium(II)chlorid-bisacetonitril, Iridium (I) -bis-(1,5-cyclooctadien)-tetrafluoroborat, Iridium-(I)-(1,5-cyclooctadien)-acetylacetonat, Iridium(I)-(1c,5c-cyclooctadien)-chlorid dimer, Platin(II)-1c,5c-cyclooctadienchlorid, Platin(II)-acetylacetonat, Ruthenium(II)-carbonyl-tris-(triphenylphosphin)-dihydrid, Ruthenium(II)-acetylacetonat.

Bei dem erfindungsgemäßen Verfahren werden Katalysatormengen von 0.001 mol% - 10 mol% verwendet. Bevorzugt werden 0.01 mol% - 5 mol% Katalysator eingesetzt.

Um den bei der Reaktion freiwerdenden Wasserstoff abzufangen, ist es für das Erzielen von hohen Ausbeuten notwendig ein Oxidationsmittel zuzusetzen. Als Oxidationsmittel sind sauerstoffenthaltende Oxidationsmittel wie N-Oxide oder Peroxide, Hypochlorit, Sauerstoff oder Luft geeignet. Besonders geeignet sind N-Oxide, Wasserstoffperoxid und Alkylperoxide.

Das erfindungsgemäße Verfahren ist nicht nur einfach durchführbar sondern liefert Amide in hoher Ausbeute bei hoher Katalysatorproduktivität und Reinheit. Darüber hinaus sind die verwendbaren Oxidationsmittel einfach recyclierbar. Dadurch ist das beschriebene Verfahren besonders für die großtechnische Amidsynthese geeignet.

Die erfindungsgemäß hergestellten Amide können unter anderem eingesetzt werden als Zwischenprodukte für Pharmazeutika und Agrochemikalien, als Bausteine für Polymere und Materialien.

### Beispiele

Die nachstehenden Beispiele dienen zur Erläuterung des erfindungsmäßigen Verfahrens, ohne es darauf zu beschränken.

### Allgemeine Arbeitsvorschrift:

0.01 bis 5mol% des Übergangsmetall-Katalysators, 0.22 mmol Base und 0.44 bis 2.2 mmol des Oxidationsmittels werden in 5 ml Lösungsmittel suspendiert. Bei Raumtemperatur gibt man 2.2 bis 6.6 mmol Amin und 2.2 bis 6.6 mmol Aldehyd hinzu. Das Reaktionsgemisch wird im Druckrohr unter Rühren 8 bis 20 Stunden bei 80 bis 140 °C erhitzt. Die Ausbeute wird durch Gaschromatographie mit Hexadekan als internem Standard ermittelt. Das Lösungsmittel wird im Vakuum entfernt und der Rückstand durch Säulenchromatographie gereingt.

### Beispiel 1:

0.0223 g [Rh(COD)₂]BF₄, 0.0304 g Kaliumcarbonat und 0.258 g N-Methylmorpholin-N-oxid werden in 5ml Toluol suspendiert. Bei Raumtemperatur gibt man 0.19 ml Morpholin und 0.45 ml Benzaldehyd sowie 0.05 ml Hexadekan als internen Standard hinzu. Das Reaktiongemisch wird im Druckrohr unter Rühren 8 Stunden bei 140 °C erhitzt. Der Ansatz wird mittels Gaschromatographie (GC) analysiert. Man findet 0.41 g 4-Benzoyl-morpholin. Das entspricht einer Ausbeute von 100 % bezüglich des eingesetzten Morpholins.

### Beispiel 2:

0.0136 g [Rh(COD)Cl]₂, 0.0304 g Kaliumcarbonat und 0.258 g N-Methylmorpholin-N-oxid werden in 5 ml Toluol suspendiert. Bei Raumtemperatur gibt man 0.43 ml Piperidin und 0.225 ml Benzaldehyd sowie 0.05 ml Hexadekan als internen Standard hinzu. Das Reaktiongemisch wird im Druckrohr unter Rühren 20 Stunden bei 140 °C erhitzt. Der Ansatz wird mittels Gaschromatographie (GC) analysiert. Man findet 0.39 g 1-Benzoyl-piperidin. Das entspricht einer Ausbeute von 93 % bezüglich des eingesetzten Benzaldehyds.

### Beispiel 3:

0.0223 g [Rh(COD)₂]BF₄, 0.0304 g Kaliumcarbonat und 0.258 g N-Methylmorpholin-N-oxid werden in 5ml THF suspendiert. Bei Raumtemperatur gibt man 0.26 ml N-Methylbutylamin und 0.45 ml Benzaldehyd sowie 0.05 ml Hexadekan als internen Standard hinzu. Das Reaktiongemisch wird im Druckrohr unter Rühren 20 Stunden bei 100 °C erhitzt. Der Ansatz wird mittels Gaschromatographie (GC) analysiert. Man findet 0.39 g N-Benzoyl-N-methylbutylamin. Das entspricht einer Ausbeute von 94 % bezüglich des eingesetzten N-Methylbutylamins.

### Weitere Beispiele:

Die in Tabelle 1 beschriebenen Umsetzungen wurden wie folgt durchgeführt: 0.0223 g [Rh(COD)₂]BF₄, 0.0304 g Kaliumcarbonat und 0.258 g N-Methylmorpholin-N-oxid werden in 5 ml des angegebenen Lösungsmittels suspendiert. Bei Raumtemperatur gibt man 2.2 mmol des angegebenen Amins und 4.4 mmol des angegebenen Aldehyds ( oder des jeweils angegebenen molaren Verhältnisses von Amin und Aldehyd) sowie 0.05 ml Hexadekan als internen Standard hinzu. Das Reaktiongemisch wird im Druckrohr unter Rühren in der angegebenen Zeit bei der angegebenen Temperatur erhitzt. Der Ansatz wird mittels Gaschromatographie (GC) analysiert. Die angegebenen Ausbeuten beziehen sich auf den jeweils im Unterschuß eingesetzten Reaktanden.

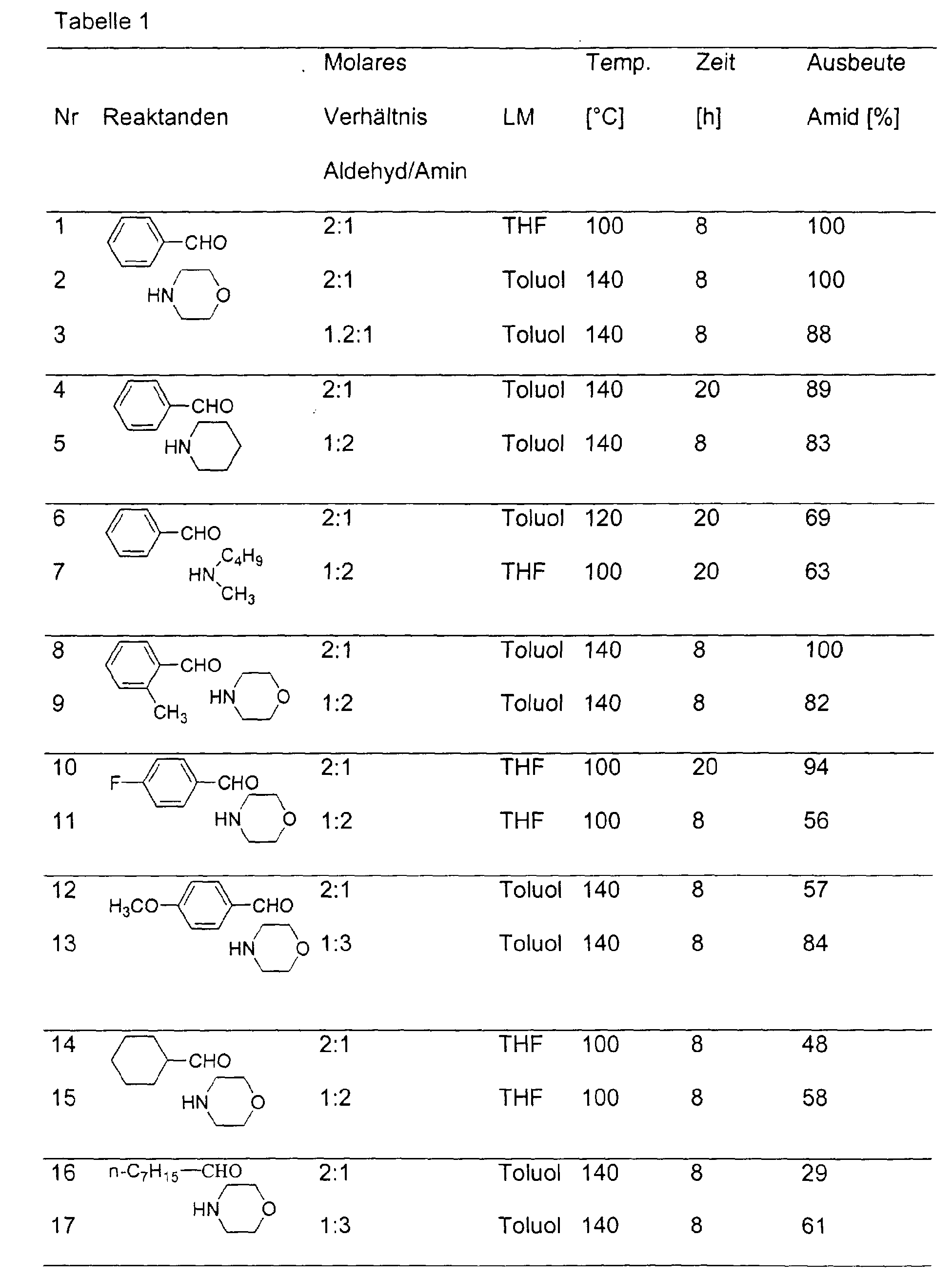

## Patentansprüche

1. Verfahren zur Herstellung von mono-, bi- oder/und polyfunktionellen Amiden der Formeln (la) oder/und (Ib),
R¹-CO-NR²R³ (Ia)
R⁴R⁵N-CO-R⁶-CO- NR²R³ (Ib)
worin
R¹ für Wasserstoff, einen (C₁-C₁₈)-Alkylrest, (C₂-C₁₈)-Alkenylrest, (C₂-C₁₈)-Alkinylrest welche verzweigt, linear oder cyclisch sein können, sowie für einen bis zu 14 C-Atome enthaltenden aromatischen Arylrest oder Heteroarylrest, enthaltend ein bis vier Heteroatome aus der Gruppe N, O oder S, steht,
wobei jeder dieser Reste neben Wasserstoffatomen und der Amidgruppe bis zu fünf weitere Substituenten tragen kann, die unabhängig voneinander (C₁-C₈)-Alkyl, O-Alkyl-(C₁-C₈), O-Aryl, OCO-Alkyl-(C₁-C₈), OCO-Aryl, O-Phenyl, Phenyl, Aryl, Fluor, Chlor, Brom, lod, OH, NO₂, SiAlkyl₃-(C₁-C₈), CN, COOH, SO₃H, NH-Alkyl-(C₁-C₈), NH-Aryl, N-Alkyl₂-(C₁-C₈), N-Aryl₂, SO₂-Alkyl-(C₁-C₆), SO₂-Aryl, SO-Alkyl-(C₁-C₆), CF₃, NHCO-Alkyl-(C₁-C₄), COO-Alkyl-(C₁-C₈), COOAryl, CONH₂, CO-Alkyl-(C₁-C₈), CO-Aryl, NHCOH, NHCOO-Alkyl-(C₁-C₄), CO-Phenyl, COO-Phenyl, CHCH-CO₂-Alkyl-(C₁-C₈), PO-Phenyl₂, POAlkyl₂-(C₁-C₄), PO₃H₂, PO(O-Alkyl-(C₁-C₆))₂, SO₃-Alkyl-(C₁-C₄) bedeuten,
wobei der Arylrest für einen fünf-, sechs- oder siebengliedrigen aromatischen oder heteroaromatischen Ring mit ein bis vier Heteroatomen aus der Gruppe N, O und S steht und wobei an den Ring weitere aromatische, heteroaromatische oder/und aliphatische Ringe mit 4 bis 16 Kohlenstoffatomen ankondensiert sein können, wobei 1 bis 8 Kohlenstoffatome durch Heteroatome aus der Gruppe N, O, S ersetzt sein können, und worin
R² bis R⁵ unabhängig voneinander Wasserstoff, Alkyl-(C₁-C₁₈), Aryl, bedeuten, wobei Alkyl und Aryl die oben genannte Bedeutung haben und bis zu 5 der oben genannten Substituenten neben Wasserstoff besitzen können, und worin
R⁶ für einen (C₁-C₁₈)-Alkylenrest, der verzweigt, linear und/oder cyclisch sein kann, sowie für einen bis zu 14 C-Atome enthaltenden aromatischen Arylenrest steht,
wobei der Alkylenrest und/oder Arylenrest neben Wasserstoffatomen und den Amidgruppen bis zu fünf weitere Substituenten tragen kann, die unabhängig voneinander (C₁-C₈)-Alkyl, O-Alkyl-(C₁-C₈), O-Aryl, OCO-Alkyl-(C₁-C₈), OCO-Aryl, O-Phenyl, Phenyl, Aryl, Fluor, Chlor, Brom, lod, OH, NO₂, SiAlkyl₃-(C₁-C₈), CN, COOH, SO₃H, NH-Alkyl-(C₁-C₈), NH-Aryl, N-Alkyl₂-(C₁-C₈), N-Aryl₂, SO₂-Alkyl-(C₁-C₆), SO₂-Aryl, SO-Alkyl(C₁-C₆), CF₃, NHCO-Alkyl-(C₁-C₄), COO-Alkyl-(C₁-C₈), COOAryl, CONH₂, CO-Alkyl-(C₁-C₈), CO-Aryl, NHCOH, NHCOO-Alkyl-(C₁-C₄), CO-Phenyl, COO-Phenyl, CHCH-CO₂-Alkyl-(C₁-C₈), PO-Phenyl₂, POAlkyl₂-(C₁-C₄), PO₃H₂, PO(O-Alkyl-(C₁-C₆))₂, SO₃-Alkyl-(C₁-C₄) bedeuten, wobei der Arylrest auch für einen fünf-, sechs- oder siebengliedrigen aromatischen oder heteroaromatischen Ring steht, wobei der heteroaromatische Ring ein bis vier Heteroatomen aus der Gruppe N, O, S enthalten kann, wobei an den Ring weitere aromatische, heteroaromatische oder/und aliphatische Ringe mit 4 bis 16 Kohlenstoffatomen, ankondensiert sein können, wobei 1 bis 8 Kohlenstoffatome durch Heteroatome aus der Gruppe N, O, S ersetzt sein können,
durch Umsetzung von Aldehyden oder/und Dialdehyden der allgemeinen Formeln (lla) oder/und (IIb)
R¹-CHO (IIa)
OHC-R⁶-CHO (IIb)
mit Aminen der allgemeinen Formel (IIIa,b),
R⁴R⁵NH (IIIa)
HNR²R³ (IIIb)
worin bei den Formeln des Typs II und III R¹ bis R⁶ die zuvor zu den Formeln des Typs (I) angegebene Bedeutung besitzen,
in Gegenwart eines Übergangsmetallkatalysators der VIII. Nebengruppe und einem Oxidationsmittel.

2. Verfahren gemäß Anspruch 1 **dadurch gekennzeichnet, daß** die Reste R² bis R⁵ für (C₁-C₈)-Alkyl oder die in Anspruch 1 beschriebenen substituierten (C₁-C₈)-Alkyle stehen.

3. Verfahren nach einem der vorhergehenden Ansprüche **dadurch gekennzeichnet, daß** als Übergangsmetallkatalysatoren Metallverbindungen enthaltend Rh, Pd, Ir, Ru, Co, Pt eingesetzt werden.

4. Verfahren nach einem der vorherigen Ansprüche **dadurch gekennzeichnet, daß** Rhodiumkatalysatoren eingesetzt werden.

5. Verfahren nach Anspruch 1 **dadurch gekennzeichnet, daß** als Katalysator Rhodium(I)-bis-(1,5-cyclooctadien)-tetrafluoroborat, Rhodium(I)-(1,5-cyclooctadien)-acetylacetonat, Rhodium(I)-(1c,5c-cyclooctadien)-chlorid dimer, Palladium(II)acetat, Palladium(II)chlorid, Lithiumtetrachloropalladat, Palladium(II)acetylacetonat, Palladium(II)chlorid-bisacetonitril, Iridium (I)-bis(1,5-cyclooctadien)-tetrafluoroborat, Iridium-(I)-(1,5-cyclooctadien)-acetylacetonat, Iridium(I)-(1c,5c-cyclooctadien)-chlorid dimer, Platin(II)-1c,5c-cyclooctadienchlorid, Platin(II)-acetylacetonat, Ruthenium(II)-carbonyl-tris-(triphenylphosphin)-dihydrid, Ruthenium(II)-acetylacetonat eingesetzt wird.

6. Verfahren nach einem der vorherigen Ansprüche **dadurch gekennzeichnet, daß** Katalysatormengen von 0.001 mol% - 10 mol% verwendet werden.

7. Verfahren nach einem der vorherigen Ansprüche **dadurch gekennzeichnet, daß** als Oxidationsmittel sauerstoffenthaltende Oxidationsmittel verwendet werden.

8. Verfahren nach einem der vorherigen Ansprüche **dadurch gekennzeichnet, daß** als Oxidationsmittel N-Oxide, Peroxide, Hypochlorit, Sauerstoff oder Luft verwendet werden.

9. Verfahren nach einem der vorherigen Ansprüche **dadurch gekennzeichnet, daß** als Oxidationsmittel Wasserstoffperoxid oder Alkylperoxide verwendet werden.

10. Verfahren nach einem der vorherigen Ansprüche **dadurch gekennzeichnet, daß** dem Reaktionsgemisch eine Base als Co-Katalysator zugesetzt wird.
